# EUROPEAN PATENT APPLICATION

(11) **EP 3 136 323 A1**
(43) Date of publication of application: **01.03.2017**
(21) Application number: 15182908.2
(22) Date of filing: 28.08.2015
(51) Int. Cl.: G06Q 10/10, A61B 5/11, G01S 19/19, G01K 1/02

(54) **APPARATUS, SYSTEM AND METHOD FOR DETERMINING SKIING RELATED INFORMATION**

(71) Applicant: Exiops Oy, 90590 Oulu (FI)
(72) Inventor: Höynälä, Marko, 90240 Oulu (FI)
(74) Representative: Suominen, Kaisa Liisa

(57) **Abstract**

Disclosed are apparatus, system and method for determining skiing related information of a skier and a surrounding of the skier. The system comprises an apparatus and a server communicably coupled to the apparatus. The apparatus comprises a body configured to be mounted on a skiing accessory. The apparatus further comprises a sensor assembly adapted to be at least partially accommodated in a recess of the body. The sensor assembly comprises a motion sensor to measure movement of the skier by measuring movement of the skiing accessory worn by the skier, a temperature sensor to remotely measure temperature of snow proximate to the skier, and a location sensor to measure location of the skier. The server is operable to collect the measured data and process the measured data to determine the skiing related information.

## Description

### TECHNICAL FIELD

The present disclosure relates generally to use of sensor technology in winter sports; and more specifically, to determining skiing related information associated with a skier and a surrounding of the skier.

### BACKGROUND

Winter sports are typically played in cold areas during winters. For example, such winter sports may include skiing, cross-country skiing, alpine skiing, snowboarding, ski jumping and the like. Generally, such winter sports include a pair of skis or a snowboard configured to be attached to the boots worn by the skier. Further, during skiing the skier uses a pair of ski poles to drive himself and to manoeuvre himself (such as maintain balance, change speed and direction). However, during snowboarding the skier uses his body mass to drive and manoeuvre himself. Therefore, in such winter sports it is preferred to know various skiing related information which may prove useful for the skier to enhance his performances, skills and safety. Generally, such skiing related information may be associated with a skier and/or a surrounding of the skier. There are conventional arrangements that measure such skiing related information.

However, such conventional systems and methods do not effectively measure the skiing related information. For example, the skiing related information may include but not be limited to a geometrical profile of a skiing track and a temperature profile of the skiing track, which is useful for application of wax and other treatments at a bottom of the skis or the snowboard. Further, the conventional systems and methods do not measure biological parameters of the skier which may prove useful from performance and safety point of view. Moreover, the conventional systems and methods do not provide any effective analysis of skier's movement related data (such as comparison of skiing styles) which may prove useful for enhancing skiing skills.

Therefore, in light of the foregoing discussion, there exists a need to overcome the aforementioned drawbacks of conventional systems and methods for measuring skiing related information.

### SUMMARY

The present disclosure seeks to provide an apparatus for measuring skiing related information associated with a skier and a surrounding of the skier.

The present disclosure also seeks to provide a system for determining skiing related information associated with a skier and a surrounding of the skier.

The present disclosure also seeks to provide a method for determining skiing related information associated with a skier and a surrounding of the skier.

In one aspect, an embodiment of the present disclosure provides a system for determining skiing related information of a skier and a surrounding of the skier, the system comprising:
- an apparatus arrangeable on the skier, the apparatus comprising a sensor assembly configured to measure movement data of the skier, temperature of snow and location of the skier; and
- a server communicably coupled to the apparatus to collect the measured data, the server being operable to at least partially process the measured data to determine the skiing related information.

In another aspect, an embodiment of the present disclosure provides an apparatus for measuring skiing related information associated with a skier and a surrounding of the skier, the apparatus comprising
- a body configured to be mounted on a skiing accessory; and
- a sensor assembly adapted to be at least partially accommodated in a recess of the body, the sensor assembly comprising:
   - a motion sensor to measure movement of the skier by measuring movement of the skiing accessory worn by the skier,
   - a temperature sensor to remotely measure temperature of snow proximate to the skier, and
   - a location sensor to measure location of the skier.

In yet another aspect, an embodiment of the present disclosure provides a method for determining skiing related information of a skier and a surrounding of the skier, the method comprising:
- measuring data associated with the skier and the surrounding of the skier using an apparatus having a sensor assembly configured to be arranged on the skier, wherein the sensor assembly is configured to measure movement data of the skier, temperature of snow and location of the skier; and
- collecting the measured data by a server communicably coupled to the apparatus, wherein the server is operable to at least partially process the measured data to determine the skiing related information.

Embodiments of the present disclosure substantially eliminate or at least partially address the aforementioned problems in the prior art, and enables effective measurement of the skiing related information.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:
- FIG. 1: is a schematic illustration of a system for determining skiing related information associated with a skier and a surrounding of the skier, in accordance with an embodiment of the present disclosure;
- FIG. 2: is a block diagram of an apparatus for measuring the skiing related information associated with the skier and the surrounding of the skier, in accordance with an embodiment of the present disclosure;
- FIG. 3: is a perspective view of the apparatus, in accordance with an embodiment of the present disclosure;
- FIG. 4: is a schematic illustration of the apparatus arranged on a skiing accessory, in accordance with an embodiment of the present disclosure;
- FIG. 5: is a schematic illustration of the apparatus arranged on the skiing accessory depicting a functional state thereof, in accordance with an embodiment of the present disclosure;
- FIG. 6: is an illustration of steps of a method for determining the skiing related information associated with the skier and the surrounding of the skier, in accordance with an embodiment of the present disclosure; and
- FIG. 7: is a schematic illustration of a skier in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In an aspect, an embodiment of the present disclosure provides an apparatus for measuring skiing related information associated with a skier and a surrounding of the skier. The apparatus comprises a body configured to be mounted on a skiing accessory. The apparatus also comprises a sensor assembly adapted to be at least partially accommodated in a recess (or a pocket) of the body. The sensor assembly comprises a motion sensor to measure movement of the skier by measuring movement of the skiing accessory worn by the skier, a temperature sensor to remotely measure temperature of snow proximate to the skier, and a location sensor to measure location of the skier.

In another aspect, an embodiment of the present disclosure provides a system for determining skiing related information of a skier and a surrounding of the skier. The system comprises an apparatus arrangeable on the skier. The apparatus comprises a sensor assembly configured to measure movement data of the skier, temperature of snow and location of the skier. The system also comprises a server communicably coupled to the apparatus to collect the measured data, the server being operable to at least partially process the measured data to determine the skiing related information.

In yet another aspect, an embodiment of the present disclosure provides a method for determining skiing related information of a skier and a surrounding of the skier. The method comprises measuring data associated with the skier and the surrounding of the skier using an apparatus having a sensor assembly configured to be arranged on the skier, wherein the sensor assembly is configured to measure movement data ofthe skier, temperature of snow and location of the skier; and collecting the measured data by a server communicably coupled to the apparatus, wherein the server is operable to at least partially process the measured data to determine the skiing related information.

The apparatus is arrangeable on the skier; particularly the apparatus comprises a body configured to be mounted on a skiing accessory. In an embodiment, the skiing accessory may include but not be limited to a skiing boot, a ski pole and a ski. Further, the body ofthe apparatus is configured to have a structural configuration, which allows the apparatus to be suitably mounted on the skiing accessory.

In an example, the body of the apparatus is configured to have a shape of a loop with a coupling mechanism that enables in coupling the body on the skiing accessory. The coupling mechanism can be a buckle and pin arrangement, a button and hole, Velcro or the like that allows adjustably coupling the apparatus to the skiing accessory. Alternatively, the body of the apparatus may be configured to have a cuboidal structure adapted to be mounted on the skiing accessory using a coupling mechanism, such as Velcro or fastening clip.

The apparatus comprises the sensor assembly adapted to be at least partially accommodated in a recess of the body. The recess may include a hollow space configured on a central portion of the body (having a shape of the loop) for partially accommodating the sensor assembly therein. The sensor assembly comprises a motion sensor, a temperature sensor and a location sensor. The sensor assembly may further be configured to measure at least one biological parameter of the skier. Specifically, the sensor assembly may thus also include a body sensor.

In one embodiment, the motion sensor, the temperature sensor, the location sensor along with other electronic components forms an electronic unit adapted to be received in recess of the body. However, when a body sensor is used, it may lie outside that electronic unit, and be arrangeable on the body of the skier to measure at least one biological parameter of the skier.

In an example, the body sensor may be worn in a finger, wrist or any suitable body part of the skier from where biological signals can be derived for measuring a biological parameter, such as a heart rate of the skier during and after the skiing. Alternatively, the body sensor may be configured to measure other biological parameters, such as body pressure and body temperature, which enables determining an overall health condition of the skier while skiing.

The motion sensor is operable to measure movement data of the skier by measuring movement of the skiing accessory worn by the skier. In an embodiment, when the apparatus is mounted on a boot (skiing accessory) the skier's movement data corresponds to the movement of the boot. Alternatively, when the apparatus is mounted on a ski or a ski pole, the skier's movement data may correspond to the movement of the ski or the ski pole.

According to an embodiment, the motion sensor may include but not be limited to at least one of an accelerometer and a gyroscope. In an example, the motion sensor may be oriented perpendicular, i.e. perpendicular to a skiing surface or along a height of the boot, for efficiently measuring the movement data of the skier.

In an embodiment, the movement data of the skier comprises movement associated with a body of the skier. The movement data of the skier in turn depends on a terrain of a skiing track and a skiing style of the skier. For example, the terrain may include irregularities in terms of skiing surface, i.e. up, down and plane surfaces constituting the skiing track. Further, the skier may have a particular skiing style that may also influence the movement data of the skier.

In an example, the motion sensor, i.e. the accelerometer measures proper acceleration (i.e. total specific g-force) that the skiing accessory (on which the apparatus is mounted) is subjected to when the skier is skiing. In an embodiment, the accelerometer may be selected from a group consisting of a one-axis accelerometer, a two-axis accelerometer and a three-axis accelerometer. Further, the gyroscope (angular rate sensor) measure change in angular position (or orientation) of the skiing accessory based on the angular momentum subjected to the skiing accessory. In an embodiment, the gyroscope may be selected from a group consisting of a one-axis angular rate sensor, a two-axis angular rate sensor and a three-axis angular rate sensor. This allows the motion sensor to be utilized to track direction and speed of the skier even with complex angular movements and high speeds.

According to an embodiment, the motion sensor data may be correlated with movement of the user to measure the movement data of the skier. For example, the movement data of the skier may include a jump taken by the skier, change in directions of the skier and a shock encountered by the skier (for example due to sudden stop) and the like. Further, the motion sensor data may be used for measuring speed data, such as an average speed, a peak speed and a lowest speed achieved by the skier. Moreover, the motion sensor data may be utilized to measure a flight distance, a flight height and a flight time when the skier jumps from a height.

As mentioned above, the temperature sensor is adapted to remotely measure temperature of snow proximate to the skier. Specifically, the temperature sensor is associated with measuring skiing related information associated with a surrounding of the skier. According to an embodiment, the sensor assembly may be also associated with other sensors capable of measuring other surrounding (or ambient) data, such as sensors capable of measuring atmospheric pressure, atmospheric temperature, oxygen content, wind speed and the like which can be collectively utilized for measuring the skiing related information associated with the surrounding of the skier.

In an embodiment, the temperature sensor of the present disclosure is an infrared temperature sensor capable of remotely measuring the temperature of the snow. Specifically, the temperature sensor is configured to measure the temperature of the snow, present on the skiing track on which the skier is skiing. It is known that any object radiates infrared energy if its temperature is above absolute zero (0 Kelvin i.e. -273.16°C or -459.69°F). Therefore, infrared signals radiated by the snow may be received by the infrared temperature sensor for remotely measuring the temperature of the snow. For example, based on an intensity of received infrared signals, the infrared temperature sensor generates an electrical signal, which in turn corresponds to a temperature value. Alternatively, the temperature of the snow may be measured by transmitting infrared signals and by measuring intensity of received reflected infrared signals.

In an embodiment, the recess comprises at least one opening allowing the infrared temperature sensor to transmit and receive infrared signals for remotely measuring the snow temperature. For example, the temperature sensor may be arranged on an edge of the electronic unit, which may be inclined at 45 degrees. Further, the edge may be configured to have an optical aperture for transmitting and receiving the infrared signals therethrough, i.e. in this embodiment, the recess comprises at least one opening allowing the infrared temperature sensor to transmit and receive infrared signals for remotely measuring the snow temperature. Moreover, the at least one opening of the recess may be configured at an angle between 20-45 degrees, for example at angle 40 degrees, with respect to a vertical axis of the skiing accessory (such as the boot). Therefore, when the electronic unit is received in the recess, the optical aperture of the electronic unit aligns with the opening of the recess for transmitting and receiving the infrared signals therethrough. Such arrangement of the infrared temperature sensor of the apparatus allows remote measurement of the temperature of the snow (proximate to the skier) without being obstructed by surface of the skis.

The location sensor is operable to measure location of the skier. In an embodiment, the location sensor is a global positioning system (GPS) sensor that measures the location of the skier. Specifically, the location sensor measures the skier's latitude and longitude along a skiing terrain, which may be accumulated to define the skiing track. Additionally, the location sensor may measure various positions of the skiers along the skiing track. For example, when the skier takes a flight, the location sensor measures position of the skier in the air and position where the skier lands.

In an embodiment, the apparatus further comprises a processing module having a memory including instructions for operating the sensors of the sensor assembly to measure sensor data. Specifically, the processing module is operable to execute instructions (or algorithm) for defining operational pattern of the sensor assembly. For example, how and when the sensor assembly should measure the data and communicate the measured data. Additionally, the processing module may be configured to at least partially process the measured data of the sensors for determining the skiing related information.

In an embodiment, the system comprises a first communicating device associated with the skier and communicably coupled to the apparatus to collect the measured data, wherein the first communicating device and the server are operable to at least partially process the measured data to determine the skiing related information.

In an embodiment, the apparatus further comprises a wireless communication module. The wireless communication module is capable of communicating the measured data of the sensor assembly to at least one of a first communicating device associated with the skier and the server communicably coupled to the apparatus. Additionally, the wireless communication module may enable establishing a communication between the body sensor and the processing module for transmitting instruction for the operation of the body sensor, and for receiving measured data from the body sensor.

In an embodiment, the first communicating device is communicably coupled to the apparatus to collect the measured data of the sensor assembly using the wireless communication module. In an example, the wireless communication module operates according to one of a short range wireless communication protocol, such as Wi-Fi or Bluetooth. Further, the first communicating device (associated with the skier) is a portable computing device, which includes but is not be limited to a smart phone, a tablet and a phablet. It may be evident that such first communicating device may be equipped with a location sensor, such as the global positioning system (GPS); in such instance the apparatus may not include the location sensor as mentioned above. Additionally, if the first communicating device also includes advanced motion sensor, i.e. the accelerometer and the gyroscope, the apparatus may not include the motion sensor as mentioned above.

In an embodiment, the first communicating device is communicably coupled to the server using a communication network. The communication network may be a wireless or a combination of wired and wireless communication network. For example, the communication network includes, but is not limited to, Local Area Networks (LANs), Wide Area Networks (WANs), Metropolitan Area Networks (MANs), Wireless LANs (WLANs), Wireless WANs (WWANs), Wireless MANs (WMANs), the Internet, second generation (2G) telecommunication networks, third generation (3G) telecommunication networks, fourth generation (4G) telecommunication networks, and Worldwide Interoperability for Microwave Access (WiMAX) networks.

In an embodiment, when the server is communicably coupled to the apparatus (instead of being communicably coupled through the first communicating device), the communication network (as mentioned above) may be utilized for establishing communication therebetween.

As mentioned above, the server is operable to at least partially process the measured data to determine the skiing related information. For example, the server may be a dedicated computer system or a plurality of computer systems communicably coupled together to process the measured data. In one embodiment, the first communicating device and the server are operable to at least partially process the measured data to determine the skiing related information. For example, the first communicating device may initially receive the measured data from which a portion of the measured data may be processed by the first communicating device and remaining portion of the measured data may be communicated to the server for processing thereof. Otherwise, the first communicating device may communicate the entire measured data to the server for the processing thereof. Additionally, the apparatus may directly communicate the entire measured data to the server for the processing thereof.

In an embodiment, the skiing related information may be associated with the skiing track. For example, the measured data of the location sensor may be at least partially processed by the first communicating device and the server is operable to determine a shape of the skiing track. Specifically, the latitudes and longitudes measured by the location sensor along a skiing terrain may be accumulated to define the shape of the skiing track.

In another embodiment, the skiing related information may be associated with a temperature of a skiing track. Specifically, the measured data of the temperature sensor may be collected and thereafter may be correlated with the location sensor data to determine a temperature profile of the skiing track. For example, the temperature sensor may measure the temperature of snow at a start point A (say -5 degree Celsius), temperatures at intermediate points B, C (say -4 and -2 degree Celsius respectively) and temperature at an end point D (say 0.5 degree Celsius). Accordingly, the temperature sensor data and the location sensor data corresponding to various locations such as the start, intermediate and end points A, B, C and D may be collated and correlated for determining the temperature profile of the overall skiing track. Further, such information (i.e. temperature profile of the overall skiing track) may be associated with weather forecast to predict future temperature profile of the skiing track. This information is useful at the time of application of wax and other treatments at the bottom of the skis or the snowboard.

In another embodiment, the skiing related information may be associated with a skiing style of the skier. Specifically, the measured data of the motion sensor may be collected and correlated with the location sensor data to determine the skiing style. For example, the measured data of the motion sensor (i.e. the movement data of the skier), such as jump taken by the skier, change in directions of the skier, the speed data, the flight data and the like, may be correlated with the location sensor data to determine the skiing style of the skier. The skiing style may be subjective in nature with respect to various skiers. Further the skiing related motion and orientation sensor related information can be analysed using an algorithm which can distinguish the classical and skating technique by utilizing the information received from the movement sensor data, particularly "pitch" and "roll" information. There are clear patterns in pitch and/or roll related measurement data which are unique for both techniques. These patterns are detectable with the mathematical algorithm.

In one embodiment, the skiing related information may be associated with a performance of the skier. Specifically, the server is configured to store the skiing related information (such as the movement data of the skier and skiing style of the skier) in a database communicably coupled to the server. Further, the server may be operable to categorize the skiing related information based on the skier. For example, the server may be operable to execute learning algorithms based on neural networks to train the server with the skiing related information for the categorization of the skiing related information based on the skier. For example, when the server is stored with the skiing related information associated with various skiers, the server may be operable to identify skiing related information associated with a particular skier with the help of the learning algorithms based on neural networks.

According to an embodiment, based on the stored skiing related information, a skier may determine and improve his skiing style. For example, based on stored skiing related information a comparison can be made between completion times for a particular skier on a particular skiing track. Thereafter, a best completion time may be selected and the skiing related information (such as the movement data of the skier and skiing style of the skier) associated with the best time may be analysed by the skier for improving his skiing style and performance. Additionally, one skier may analyze the skiing related information (such as the movement data of the skier and skiing style of the skier) of other better skiers for improving his skiing style and performance.

In yet another embodiment, the skiing related information may be associated with health of the skier. For example, the measured data of the body sensor may be collected and correlated with the location sensor data to determine the health of the skier while skiing on a particular skiing track. The body sensor data (i.e. measured biological parameters such as heart rate, body temperature and pressure) may be useful in determining a required fitness level of a skier.

In an embodiment, the system further includes a second communicating device communicably coupled to the server for providing the determined skiing related information to at least the skier and a user associated with the skier. The second communicating device may be communicably coupled to the server using the communication network, as mentioned above. Further, the second communicating device may be a smart phone, a tablet, a phablet, a laptop, a desktop and the like.

In an embodiment, the method may thus further comprise storing the skiing related information and categorizing the skiing related information based on the skier. According to another embodiment, the method may comprise providing the skiing related information to at least the skier and a user associated with the skier.

In an embodiment, the user associated with the skier may be a coach responsible for training the skier for improving the skiing performance of the skier. Otherwise, the user may any person interested in skiing and/or the skier himself.

According to an embodiment, the determined skiing related information may be presented in the form of maps, graphs, line charts, pie-charts and the like. For example, the temperature profile of a skiing track may be shown as a map depicting various temperature values associated with a skiing track. Similarly, the movement data of the skier may be shown as a graph depicting, for example, his speeds at different instants of time.

According to an embodiment, the system of the present disclosure includes a single apparatus to be mounted on a single skiing accessory, such as the boot. Alternatively, the system may include a pair of apparatuses adapted to be mounted on both boots. In such instance, although there may be two apparatuses, there would be one body sensor.

The present disclosure provides an apparatus, a system and a method for determining skiing related information associated with a skier and surrounding of the skier. Specifically, the present disclosure enables in measuring temperature of snow, which is useful for application of wax and other treatments to a bottom of the skis or the snowboard. This is turn may influence skiing ability of the skier. For example, such treatment may provide traction for forward movement but also break loose and glide after a certain speed is reached by the skier. Further, the skiing related information may be utilized for identifying challenges associated with skiing tracks and health of the skier. Moreover, the present disclosure also enables in effective analysis of the skiing related information which may prove useful for enhancing skiing skills (or performance) of the skier. Specifically, the skiing related information may be help coaches and trainers while training experienced skiers or beginners. For example, the skiing related information may be utilized to guide the skiers to change their skiing style in order to improve their skiing performance.

### DETAILED DESCRIPTION OF THE DRAWINGS

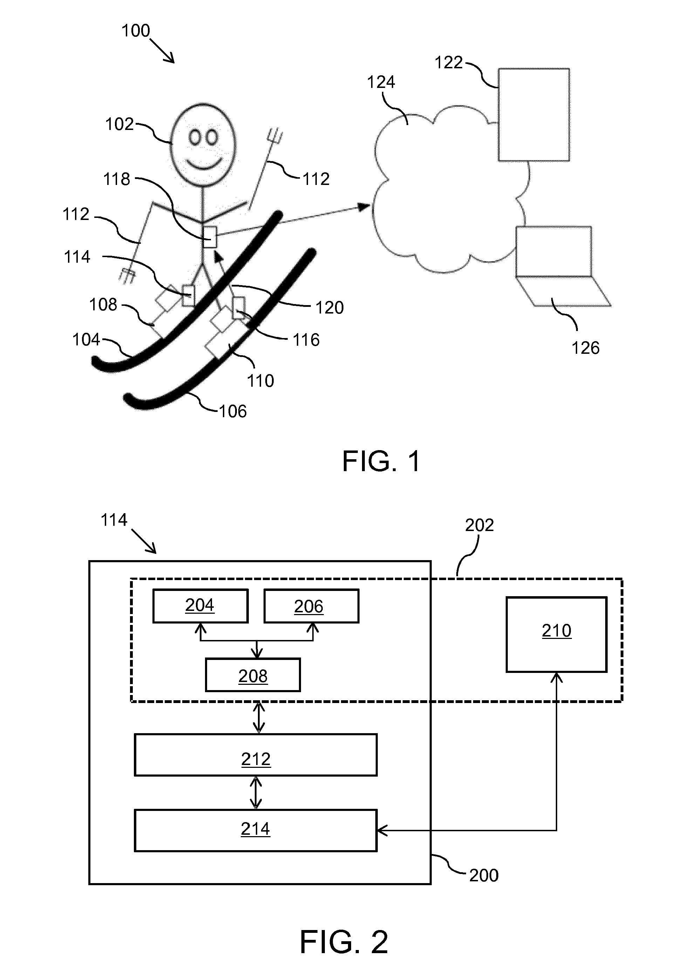

Referring to FIG. 1, illustrated is a schematic illustration of a system **100** for determining skiing related information associated with a skier **102** and a surrounding of the skier **102,** in accordance with an embodiment of the present disclosure. As shown, the skier **102** is skiing with the help of skiing accessory, such as skies **104** and **106.** The ski **104** is worn on a right foot of the skier **102** with the help of a ski boot **108.** Similarly, the ski **106** is worn on a left foot of the skier **102** with the help of a ski boot **110.** Moreover, the skier **102** is using skiing accessory, such as a pair of ski poles **112.**

The system **100** includes an apparatus arrangeable on the skier. As shown, the apparatuses **114** and **116** are mounted the ski boots **108,110,** respectively. The apparatuses **114, 116** are communicably coupled to a first communicating device **118** associated with the skier **102.** For example, as shown, the apparatus **116** is communicably coupled to the first communicating device **118** using a wireless communication link **120.** The first communicating device **118** is accordingly configured to receive data from the apparatuses **114, 116.** The first communicating device **118** is further communicably coupled to a server **122** via a communication network **124.** The apparatuses **114, 116** are configured to measure movement data of the skier, temperature of snow, location of the skier and biological parameter of the skier. Thereafter, the measured data is communicated to the first communicating device **118,** such that the first communicating device **118** and the server **122** are operable to partially process the measured data to determine the skiing related information.

The system **100** also includes a second communicating device **126** communicably coupled to the server **122** for providing the determined skiing related information to at least the skier **102** and a user associated with the skier **102.** As shown, the second communicating device **126** is communicably coupled to the server **122** via the communication network **124.**

Referring now to FIG. 2, illustrated is a block diagram of an apparatus, such as the apparatus **114,** for measuring skiing related information associated with the skier **102** (shown in FIG. 1) and the surrounding of the skier **102,** in accordance with an embodiment of the present disclosure. The apparatus **114** includes a body **200** configured to be mounted on the skiing accessory (such as the ski boot **108,** as shown in FIG. 1). The apparatus **114** also includes a sensor assembly **202** adapted to be at least partially accommodated in the body **200.** As shown, the sensor assembly **202** includes a motion sensor **204,** a temperature sensor **206** and a location sensor **208,** accommodated in the body **200.** The sensor assembly **202** also includes a body sensor **210,** outside the body **200,** and arrangeable on a body of the skier **102** to measure at least one biological parameter of the skier **102.**

The motion sensor **204** is configured to measure movement of the skier **102** by measuring movement of the skiing accessory (such as the ski boot **108,** as shown in FIG. 1) worn by the skier **102.** Specifically, the motion sensor **204** is configured to measure movement of the skiing accessory on which the apparatus **114** is arranged. Further, the temperature sensor **206** is configured to remotely measure the temperature of snow proximate to the skier **102.** Moreover, the location sensor **208** is configured to measure a location of the skier **102.**

As shown, the apparatus **114** further includes a processing module **212** having a memory including instructions for operating the sensors **204, 206, 208** and **210** of the sensor assembly **202** to measure sensor data. The apparatus **114** also includes a wireless communication module **214** to communicate the measured data of the sensor assembly **202** to at least one of the first communicating device **118** (shown in FIG. 1) associated with the skier **102** and the server **122** (shown in FIG. 1) communicably coupled to the apparatus **114.** The wireless communication module **214** is also shown communicably coupled to the body sensor **210** for providing operating instructions and to receive measured data.

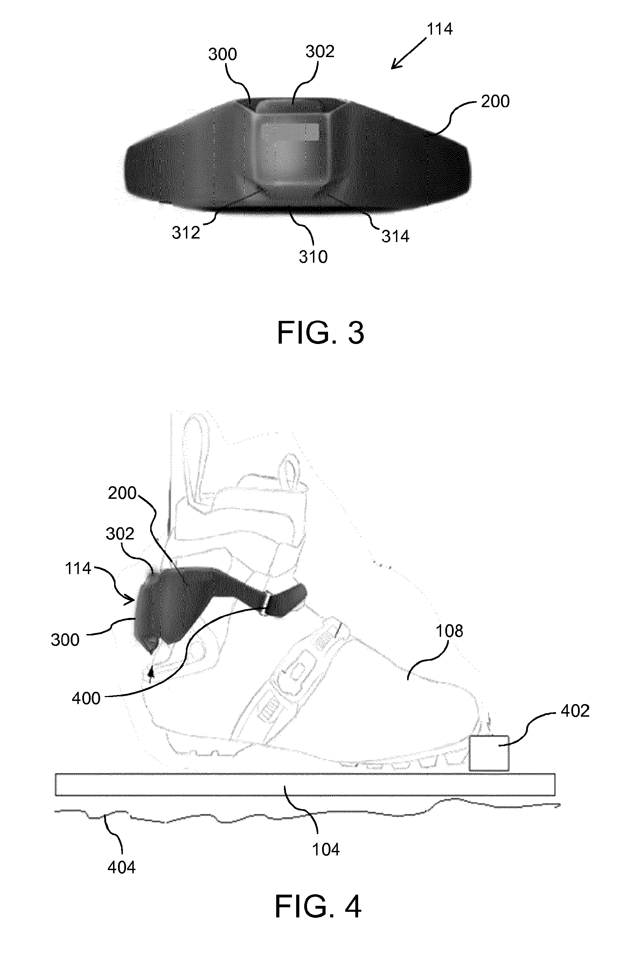

The sensor assembly **202** (excluding the body sensor **210**) along with the processing module **212** and the wireless communication module **214** form a electronic unit adapted to be received in a recess of the body **200,** which is explained in conjunction with FIG. 3.

Referring now to FIG. 3, illustrated is a perspective view of the apparatus **114,** in accordance with an embodiment of the present disclosure. As shown, the apparatus **114** includes the body **200** (for example having a loop shape) and the sensor assembly **202** (shown in FIG. 2) partially accommodated in the body **200.** Specifically, the body **200** includes a recess **300** (for partially accommodating the sensor assembly **202**), i.e. accommodating the electronic unit **302** (which includes the motion sensor **204,** the temperature sensor **206,** the location sensor **208** along with the processing module **212** and the wireless communication module **214**). The recess **300** is configured on a central portion **310** of the body **200** and configured to accommodate the electronic unit **302** therein.

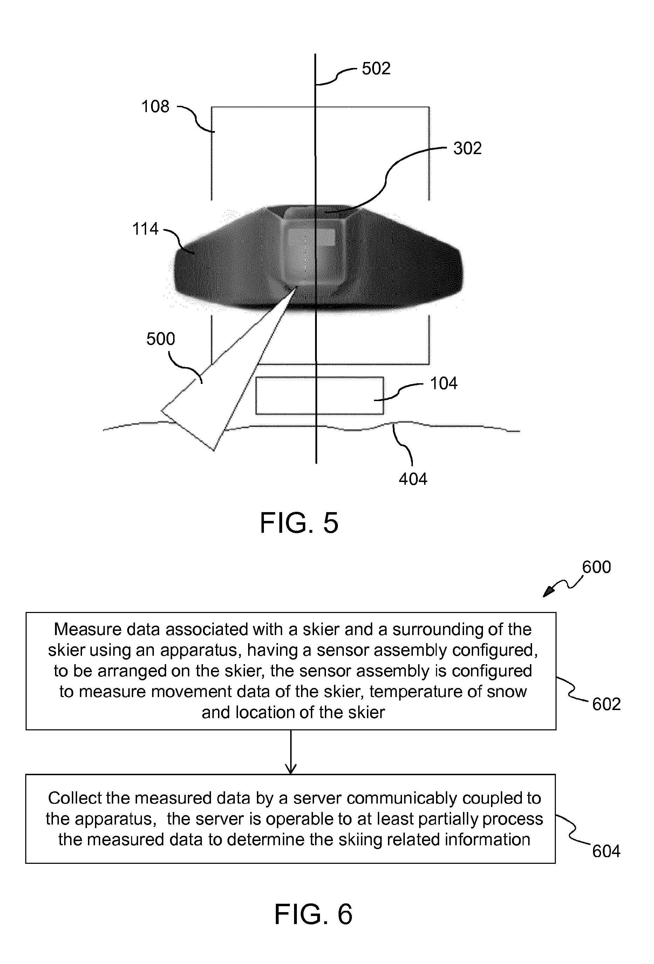

The temperature sensor **206** (shown in FIG. 2) is an infrared temperature sensor for remotely measuring temperature of the snow. As shown, the recess **300** includes at least one opening, such as openings **312, 314** for transmitting and receiving infrared signals therethrough. Further, the temperature sensor **206** of the electronic unit **302** is arranged on an edge (not shown), which is inclined at 45 degrees, of the electronic unit **302.** The edge is configured to have an optical aperture for transmitting and receiving the infrared signals therethrough. Specifically, when the electronic unit **302** is received in the recess **300,** the optical aperture of the electronic unit **302** aligns with an opening, such as the openings **312,** for transmitting and receiving the infrared signals. Such arrangement of the infrared temperature sensor **206** of the apparatus **114** allows remote measurement of the temperature of the snow proximate to the skier **102** (particularly to the skis **104, 106,** shown in FIG. 1) without being obstructed by surface of the skis **104, 106,** which is explained in conjunction with FIGS. 4 and 5.

Referring now to FIG. 4, illustrated is a schematic illustration of the apparatus **114** arranged on the skiing accessory, such as the ski boot **108,** in accordance with an embodiment of the present disclosure. As shown, the body **200** of the apparatus **114** is configured to have the loop shape with a buckle mechanism **400** for attaching (or arranging) the apparatus **114** on the ski boot **108.** Further, the ski boot **108** is mounted on the ski **104** using a ski boot anchor **402.** Moreover, the electronic unit **302** is shown accommodated in the recess **300** of the body **200** for measuring temperature of snow **404,** without being obstructed by a surface of the ski **104,** which is further explained in conjunction with FIG. 5.

Referring now to FIG. 5, illustrated is a schematic illustration of the apparatus **114** arranged on the skiing accessory (i.e. the ski boot **108**) depicting a functional state thereof, in accordance with an embodiment of the present disclosure. Specifically, the infrared temperature sensor **206** (shown in FIG. 2) of the electronic unit **302** is arranged at an angle, about 20 to 45 degrees, with respect to an axis **502** along the ski boot **108.** This helps in receiving (and/or transmitting) the infrared signals **500** by the temperature sensor **206** (shown in FIG. 2) for measuring the temperature of the snow **404** without being obstructed by the surface of the ski **104.**

Referring now to FIG. 6, illustrated are steps of a method **600** for determining skiing related information of a skier and a surrounding of the skier, in accordance with an embodiment of the present disclosure. Specifically, the method **600** illustrates steps involved in the operation of the system **100,** explained in conjunction with the FIGS. 1-5, for determining skiing related information the skier and the surrounding of the skier.

At step **602,** data associated with the skier and the surrounding of the skier is measured using an apparatus, having a sensor assembly, configured to be arranged on the skier. The sensor assembly is configured to measure movement data ofthe skier, temperature of snow and location of the skier.

At step **604,** the measured data is collected by a server communicably coupled to the apparatus. The server is operable to at least partially process the measured data to determine the skiing related information.

The steps **602** to **604** are only illustrative and other alternatives can also be provided where one or more steps are added, one or more steps are removed, or one or more steps are provided in a different sequence without departing from the scope of the claims herein. For example, the method **600** further includes collecting the measured data by a first communicating device associated with the skier and communicably coupled to the apparatus. Further, the first communicating device and the server are operable to process the measured data to determine the skiing related information. Also, the method **600** includes providing the skiing related information, by a second communicating device communicably coupled to the server, to at least the skier and a user associated with the skier. Moreover, the method **600** further includes storing the skiing related information, and categorizing the skiing related information based on the skier.

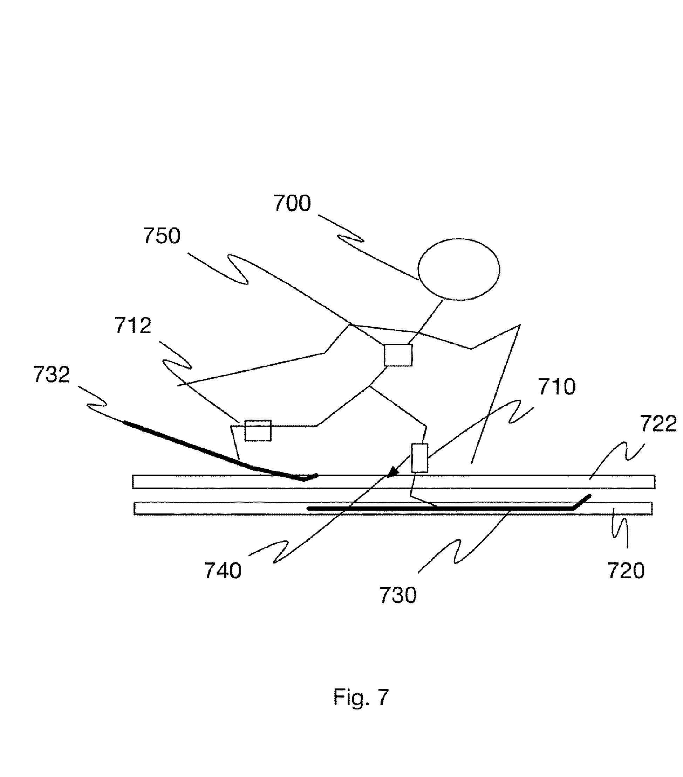

According to further embodiment, a skier **700** of FIG. 7 is skiing on ski track consisting of grooves **720** and **722** with skis **730** and **732.** The skier **700** has apparatus **710** and **712** attached to right and left foot respectively. Both apparatus **710** and **712** are commutability coupled to a first communication device **750** and/or to each other's. The information related to relative position of the apparatus **710** in respect to **712** can be used to determine whether the measurement of the snow temperature with apparatus **710** is made to the groove **722** or not. The relative position can be measured with motion sensors or location sensors or by measuring for example received signal strength indication (RSSI) from first apparatus **710** to second apparatus **712** or vice versa. Additionally the relative position can be measured using orientation and motion sensors of the apparatuses **710** and **712.** For example one can measure orientation of a feet with sensor **710,** in FIG.7 the sensor **710** would indicate that the foot is parallel to the ski track and the sensor **712** would indicate that the foot is tilted back wards (skier **700** is kicking back). This could be used to determine if the temperature measurement would result on measuring temperature of the snow or the ski **732.** Additionally phase difference between measured movement and orientation data from sensors **710** and **712** can be used to determine relative ski positions.

Further when the skier **700** kicks his left foot, the ski **732** moves backwards as indicated in the FIG. 7. As soon as the distance between the apparatus **710** and the apparatus **712** is sufficient i.e. at least length of the ski **732** in front of the ski boot, the measurement **740** of left ski groove **722** can be made with apparatus **710** attached to right foot. Further when the relative distance between two apparatuses **710** and **712** is shorter, measurement of surface temperature of left ski **732** can be made with apparatus **710** and vice versa. Tilting of infrared sensor of the apparatus **710** with a sufficient angle with respect to the foot is needed to enable measurement of left groove **722.** Tilting of infrared sensor ofthe apparatus **712** with sufficient angle in respect to the foot is needed to enable measurement of left groove **720.** Based on these embodiments, the apparatus **710** and **712** can be configured to be tilted with sufficient angles.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A system for determining skiing related information of a skier and a surrounding of the skier, the system comprising:
- an apparatus arrangeable on the skier, the apparatus comprising a sensor assembly configured to measure movement data of the skier, temperature of snow and location of the skier; and
- a server communicably coupled to the apparatus to collect the measured data, the server being operable to at least partially process the measured data to determine the skiing related information.

2. A system according to claim 1, wherein the sensor assembly is further configured to measure at least one biological parameter of the skier.

3. A system according to claim 1 or 2, further comprising a first communicating device associated with the skier and communicably coupled to the apparatus to collect the measured data, wherein the first communicating device and the server are operable to at least partially process the measured data to determine the skiing related information.

4. A system according to claim 3, further comprising a second communicating device communicably coupled to the server for providing the determined skiing related information to at least the skier and a user associated with the skier.

5. A system according to claim 3 or 4, wherein the apparatus further comprises
- a processing module having a memory including instructions for operating the sensors of the sensor assembly to measure sensor data; and
- a wireless communication module to communicate the measured data of the sensor assembly to at least one of the first communicating device associated with the skier and the server communicably coupled to the apparatus.

6. An apparatus for measuring skiing related information associated with a skier and a surrounding of the skier, the apparatus comprising
- a body configured to be mounted on a skiing accessory; and
- a sensor assembly adapted to be at least partially accommodated in a recess of the body, the sensor assembly comprising:
- a motion sensor to measure movement of the skier by measuring movement of the skiing accessory worn by the skier,
- a temperature sensor to remotely measure temperature of snow proximate to the skier, and
- a location sensor to measure location of the skier.

7. An apparatus according to claim 6, wherein the sensor assembly further comprises a body sensor arrangeable on the body of the skier to measure at least one biological parameter of the skier.

8. An apparatus according to claim 6 or 7, further comprising
- a processing module having a memory including instructions for operating the sensors of the sensor assembly to measure sensor data; and
- a wireless communication module to communicate the measured data of the sensor assembly to at least one of a first communicating device associated with the skier and a server communicably coupled to the apparatus.

9. An apparatus according to any of the claims 6-8, wherein the temperature sensor is an infrared temperature sensor for remotely measuring the temperature of the snow.

10. An apparatus according to claim 9, wherein the recess comprises at least one opening allowing the infrared temperature sensor to transmit and receive infrared signals for remotely measuring the snow temperature.

11. A method for determining skiing related information of a skier and a surrounding of the skier, the method comprising:
- measuring data associated with the skier and the surrounding of the skier using an apparatus having a sensor assembly configured to be arranged on the skier, wherein the sensor assembly is configured to measure movement data of the skier, temperature of snow and location of the skier; and
- collecting the measured data by a server communicably coupled to the apparatus, wherein the server is operable to at least partially process the measured data to determine the skiing related information.

12. A method according to claim 11, further comprising collecting the measured data by a first communicating device associated with the skier and communicably coupled to the apparatus, wherein the first communicating device and the server are operable to at least partially process the measured data to determine the skiing related information.

13. A method according to claim 11 or 12, further comprising providing the skiing related information by a second communicating device communicably coupled to the server, to at least the skier and a user associated with the skier.

14. A method according to any of the claims 11-13, further comprising
- storing the skiing related information, and
- categorizing the skiing related information based on the skier.
